# EUROPEAN PATENT APPLICATION

(11) **EP 1 535 547 A1**
(43) Date of publication of application: **01.06.2005**
(21) Application number: 03766749.0
(22) Date of filing: 05.08.2003
(51) Int. Cl.: A47G 9/10, A47G 27/02, A47G 9/02, F24F 1/02, F24F 5/00

(54) **WIND PASSAGE FOR DEHUMIDIFYING APPARATUS**

(30) Priority: 06.08.2002 JP 2002228234
(71) Applicant: Seft Development Laboratory Co. Ltd., Urawa-shi, Saitama 336-0031 (JP)
(72) Inventor: ICHIGAYA, Hiroshi, SEFT DEVELOPMENT LABORATORY CO., Minami-ku, Saitama-shi, Saitama 336-0031 (JP)
(74) Representative: Kador & Partner
(86) International application number: PCT/JP2003/009937
(87) International publication number: WO 2004/012564

(57) **Abstract**

The invention provides a dehumidifier air passage being capable of providing a sufficient effect of dehumidification and being excellent in mass productivity. A dehumidifier air passage 10 is used in a dehumidifier for removing moisture by making the outside air flow in the vicinity of the surface of a human body and the like, and comprises a nearly flat spacer 11 and a moisture transmittable sheet 21. The spacer 11 is used for supporting the moisture transmittable sheet 21 and securing a space through which air flows, and has a nearly flat base 15 and a plurality of long and narrow projections 16. Each of the projections 16 is formed so that the face of it facing the moisture transmittable sheet 21 is at most 50 % in area of the face being in contact with the base 15. A foamed plastic material is used as a material for the spacer 11. The spacer 11 is manufactured by cutting a foamed plastic material or by molding a foamed plastic material.

## Description

### TECHNICAL FIELD

The present invention relates to a dehumidifier air passage to be used in a dehumidifier such as a dehumidifying cushion, a dehumidifying mat, a dehumidifying mattress, dehumidifying clothes and the like for removing moisture by making the outside air flow in the vicinity of the surface of a human body and the like.

### BACKGROUND ART

Up to now a cane cushion has been often used in a hot season such as summer and the like. Such a cane cushion is made by knitting cane, the meshes of which are knitted with large stitches. Due to this, air is easy to naturally flow through the inside of such a cushion. When a person is seated on such a cushion, the moisture exhaled from his or her hips is transmitted into the cushion and is carried off to the outside by air flowing inside the cushion. The moisture around the hips can be removed due to this. Such a cane cushion supports human hips and performs a role as an air passage for making air flow inside the cane cushion. Therefore, it is possible to realize a dehumidifier by using such an air passage and making air forcibly flow through the air passage.

By the way, in a dehumidifier as described above, what structure is used as a dehumidifier air passage has a large influence on an effect of dehumidification. And practically, it is desirable that a dehumidifier air passage is excellent in mass productivity.

### DISCLOSURE OF THE INVENTION

The present invention has been performed on the basis of the above-mentioned circumstances and aims at providing a dehumidifier air passage being capable of providing a sufficient effect of dehumidification and being excellent in mass productivity.

The invention according to claim 1 for achieving the above-mentioned object provides a dehumidifier air passage to be used in a dehumidifier, comprising a moisture transmittable member provided at a side to be in contact with a living thing and a nearly flat spacer for supporting said moisture transmittable member and for securing a space through which air flows, said dehumidifier carrying off the moisture transmitted into said space through said moisture transmittable member from the surface of said living thing by means of air flowing through said space, wherein said spacer has a nearly flat base and a plurality of projections and is obtained by cutting a foamed plastic material or by molding a foamed plastic material, and said dehumidifier air passage has a force distributing means for distributing and applying a force applied from said living thing to said spacer, said force distributing means being provided between said moisture transmittable member and said spacer.

The invention according to claim 2 for achieving the above-mentioned object provides a dehumidifier air passage to be used in a dehumidifier, comprising a moisture transmittable member provided at a side to be in contact with a living thing and a nearly flat spacer for supporting said moisture transmittable member and for securing a space through which air flows, said dehumidifier carrying off the moisture transmitted into said space through said moisture transmittable member from the surface of said living thing by means of air flowing through said space, wherein said spacer has a nearly flat base and a plurality of projections and is obtained by forming a plurality of depressions in a plastic film by means of a molding method and then stuffing up each of said depressions with a pressure-resistive material.

The invention according to claim 3 for achieving the above-mentioned object provides a dehumidifier air passage to be used in a dehumidifier, comprising a moisture transmittable member provided at a side to be in contact with a living thing and a nearly flat spacer for supporting said moisture transmittable member and for securing a space through which air flows, said dehumidifier carrying off the moisture transmitted into said space through said moisture transmittable member from the surface of said living thing by means of air flowing through said space, wherein said spacer has a nearly flat base and a plurality of projections and is obtained by joining said plurality of projections onto said base.

The invention according to claim 4 for achieving the above-mentioned object provides a dehumidifier air passage to be used in a dehumidifier, comprising a moisture transmittable member provided at a side to be in contact with a living thing and a nearly flat spacer for supporting said moisture transmittable member and for securing a space through which air flows, said dehumidifier carrying off the moisture transmitted into said space through said moisture transmittable member from the surface of said living thing by means of air flowing through said space, wherein said spacer is obtained by molding a meshed member.

The invention according to claim 5 for achieving the above-mentioned object provides a dehumidifying clothes air passage to be used in dehumidifying clothes, comprising a nearly flat spacer for securing a space through which air flows between a human body or underwear and a clothes material, said dehumidifying clothes carrying off the moisture which has come into said space from the surface of said human body by means of air flowing through said space, wherein said spacer is obtained by molding a meshed member.

The invention according to claim 12 for achieving the above-mentioned object provides a dehumidifier air passage to be used in a dehumidifier, comprising a moisture transmittable mat provided at a side to be in contact with a living thing and a nearly flat spacer for supporting said moisture transmittable mat and for securing a space through which air flows, said dehumidifier carrying off the moisture transmitted into said space through said moisture. transmittable mat from the surface of said living thing by means of air flowing through said space, wherein said spacer has a nearly flat base and a plurality of rail-shaped projections formed in parallel with one another on said base and is obtained by an extrusion molding method, and said dehumidifier air passage has a force distributing means for distributing and applying a force applied from said living thing to said spacer, said force distributing means being provided between said spacer and said moisture transmittable mat.

The invention according to claim 13 for achieving the above-mentioned object provides a dehumidifier air passage to be used in a dehumidifier, comprising a moisture transmittable mat provided at a side to be in contact with a living thing and a nearly flat spacer for supporting said moisture transmittable mat and for securing a space through which air flows, said dehumidifier carrying off the moisture transmitted into said space through said moisture transmittable mat from the surface of said living thing by means of air flowing through said space, wherein said moisture transmittable mat has a thermal insulation ability and the air content of said moisture transmittable mat is at least 0.05 cc per area of 1 cm² in a plane nearly perpendicular to the direction of thickness of said spacer.

### BRIEF DESCRIPTION OF THE DRAWINGS

Fig. 1a is a schematic plan view of a dehumidifying cushion being a dehumidifier to which a dehumidifier air passage according to a first embodiment of the present invention is applied, and Fig. 1b is a schematic sectional view of the dehumidifying cushion seen from the direction of arrow A-A.
Fig. 2a is a schematic plan view of a spacer to be used in a dehumidifier air passage according to the first embodiment, Fig. 2b is a schematic side view of the spacer, and Fig. 2c is a schematic sectional view of the spacer seen from the direction of arrow B-B.
Fig. 3 is a figure for explaining an external shape of the spacer.
Fig. 4a is a schematic plan view of a dehumidifying mat being a dehumidifier to which a dehumidifier air passage according to a second embodiment of the present invention is applied, and Fig. 4b is a schematic sectional view of the dehumidifying mat seen from the direction of arrow C-C.
Fig. 5 is a figure showing an example of a spacer to be used in a dehumidifier air passage according to the second embodiment.
Fig. 6 is a figure showing an example of a moisture transmittable sheet and a force distributing means to be used in a dehumidifier air passage according to the second embodiment.
Fig. 7 is a figure for explaining another spacer to be used in a dehumidifier air passage according to the second embodiment.
Fig. 8a is a schematic plan view of another spacer to be used in a dehumidifier air passage according to the second embodiment, and Fig. 8b is a schematic side view of this spacer.
Fig. 9a is a schematic plan view of a dehumidifying mattress being a dehumidifier to which a dehumidifier air passage according to a third embodiment of the present invention is applied, and Fig. 9b is a schematic sectional view of the dehumidifying mattress seen from the direction of arrow D-D.
Fig. 10a is a schematic plan view of a spacer to be used in a dehumidifier air passage according to the third embodiment, and Fig. 10b is a schematic side view of this spacer.
Fig. 11a is a schematic plan view of a spacer to be used in an air passage for dehumidifying clothes, and Fig. 11b is a schematic side view of this spacer.

### BEST MODE FOR CARRYING OUT THE INVENTION

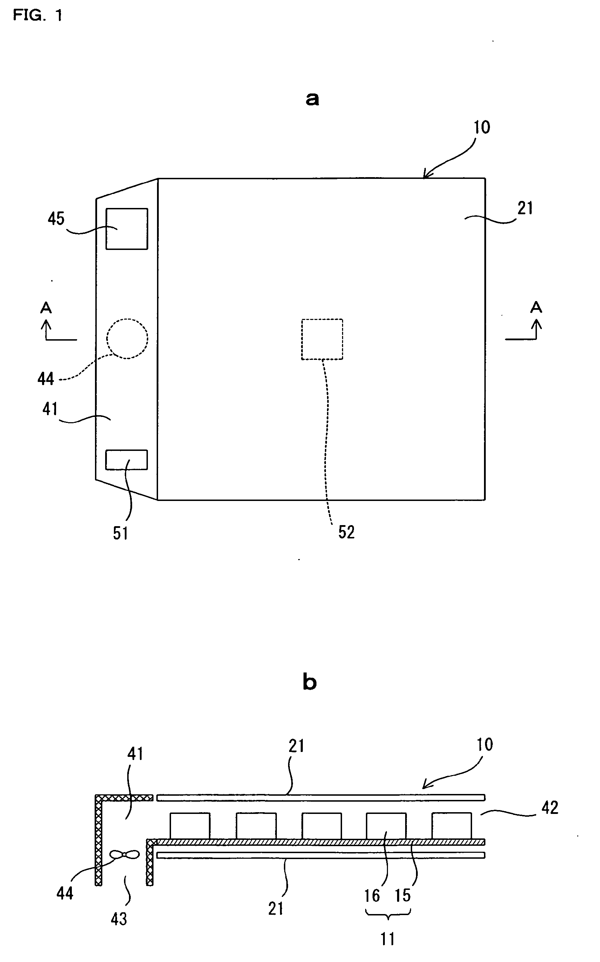

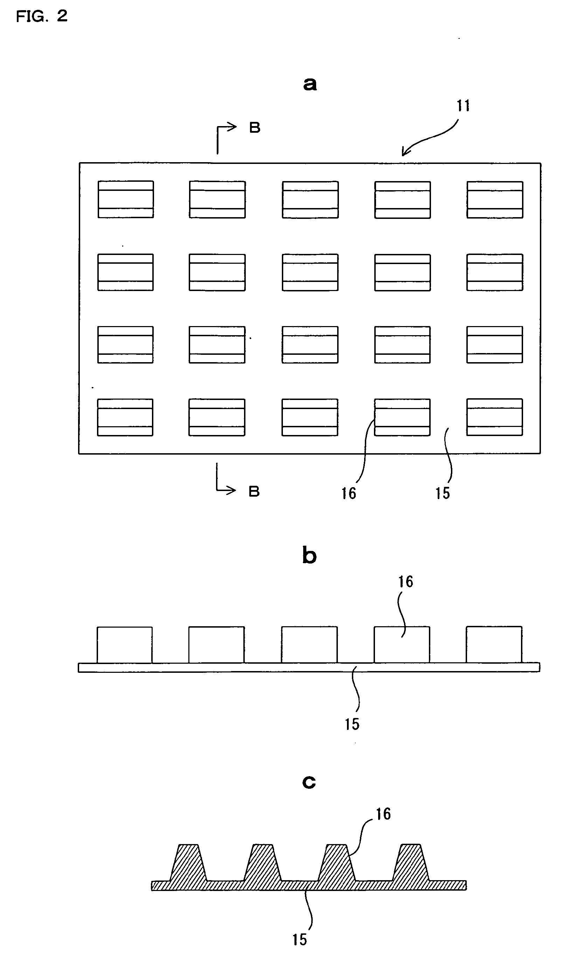

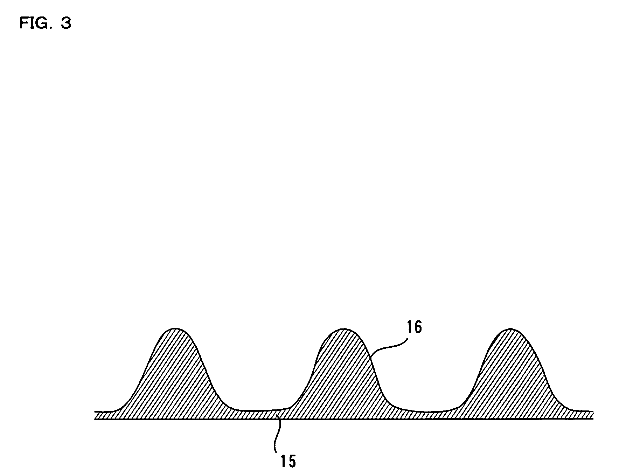

A dehumidifier air passage according to a first embodiment of the present invention is described in the following. Fig. 1a is a schematic plan view of a dehumidifying cushion being a dehumidifier to which a dehumidifier air passage according to the first embodiment of the present invention is applied, Fig. 1b is a schematic sectional view of the dehumidifying cushion seen from the direction of arrow A-A, Fig. 2a is a schematic plan view of a spacer to be used in a dehumidifier air passage according to the first embodiment, Fig. 2b is a schematic side view of the spacer, Fig. 2c is a schematic sectional view of the spacer seen from the direction of arrow B-B, and Fig. 3 is a figure for explaining an external shape of the spacer.

A dehumidifying cushion shown in Fig. 1 is used as a dehumidifier for removing the moisture coming out from the surface of the hips of a person being seated on it. In the first embodiment, the case of using such a dehumidifying cushion placed on a chair is considered. It is a matter of course that such a cushion can be also used being placed on a bench, a sofa or the like.

As shown in Fig. 1, this dehumidifying cushion comprises a dehumidifier air passage 10 being nearly in the shape of a square, a connecting passage 41, an air inlet 42, an air outlet 43, a DC fan 44 as a blowing means, a battery 45, a main switch 51 and a pressure switch 52.

As shown in Fig. 1, the dehumidifier air passage 10 has a nearly flat spacer 11 and a moisture transmittable sheet (moisture transmittable member) 21. In Fig. 1b, the right side face of the dehumidifier air passage 10 is the air inlet 42 for taking in the external air into the said air passage 10.

The spacer 11 is used for supporting the moisture transmittable sheet 21 and securing a space through which air flows. As shown in Figs. 1 and 2, this spacer 11 has a nearly flat base 15 and a plurality of long and narrow projections 16. Each of the projections 16 is arranged regularly so that the longitudinal direction of it is directed to a specified direction. These projections 16 are physically and continuously connected to one another through the nearly flat base 15. The space between adjacent projections 16 becomes a space through which air flows.

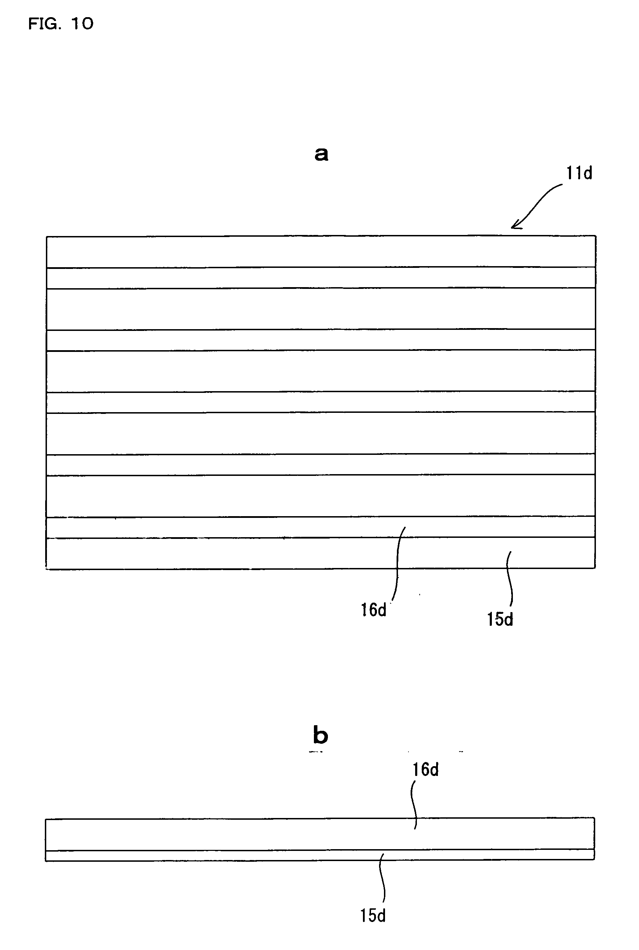

Since such a spacer 11 can be thought to be obtained by dividing each of long rail-shaped projections into plural parts, it is called a spacer of "a divided rail type". On the contrary, a spacer having a plurality of long rail-shaped projections arranged in parallel with one another is called a spacer of "a continuous rail type". For example, a spacer shown in Fig. 10 is of a continuous rail type. In the first embodiment, a spacer of a continuous rail type may be used in place of a spacer of a divided rail type 11. However, a spacer of a continuous rail type is poor in flexibility along the longitudinal direction of the projections depending on its material. The spacer of a divided rail type 11 has been improved in this point and further has an advantage of making it possible to make air flow not only in the longitudinal direction of the projections but also in a direction perpendicular to the said longitudinal direction.

Foamed plastic, for example, foamed urethane is used as a material for the spacer 11. Due to this, such a spacer 11 is characterized by being light in weight and additionally excellent in flexibility.

This spacer 11 can be manufactured by a method as shown in the following. A first method is a method of cutting a foamed plastic material. That is to say, a foamed plastic material having a thickness obtained by adding the thickness of the base 15 and the height of the projections 16 to each other is prepared. And the spacer 11 can be easily manufactured by cutting such a foamed plastic material into a specified shape. A second method is a method of thermally press-forming a foamed plastic material. That is to say, a nearly flat foamed plastic material is placed between two metal molds. And the spacer can be easily manufactured by pressing the two metal molds against each other as heating them. In case of using a spacer of a continuous rail type as the spacer 11, the spacer 11 can be easily manufactured also by extrusion-molding a foamed plastic material.

In the first embodiment, as shown in Fig. 2c, each of the projections 16 is formed so that the sectional shape of it when it is cut by a plane perpendicular to the longitudinal direction of it is a nearly tapered shape. Exactly speaking, each of the projections 16 is formed so that its face being opposite to the moisture transmittable sheet 21 is at most 50 % in area of the face to be in contact with the base 15. Using a spacer 11 having such projections 16 stands to reason in the following points. That is to say, firstly, since a projection 16 is structurally wider at the lower side than at the upper side, the projection 16 can be more enhanced in strength. Secondly, the contact area between each projection 16 and the moisture transmittable sheet 21 can be made smaller. If the said contact area is made larger, the contacting portion is surely filled with moisture but such a phenomenon does not occur in the first embodiment. Thirdly, since a space around the upper part of the spacer 11 is larger in comparison with a space around the lower part of it, it is possible to increase the quantity of air flowing near the moisture transmittable sheet 21. Due to this, the effect of dehumidification can be enhanced. And the sectional shape of a projection 16 perpendicular to the longitudinal direction of it is not limited to a nearly tapered shape. For example, the projections 16 may be in a nearly corrugated shape as shown in Fig. 3.

In Fig. 1a, the moisture transmittable sheet 21 wraps the obverse and reverse faces and the upper and lower side faces of the spacer 11 in it. A person results in being seated on the moisture transmittable sheet 21 provided on the upper face of the spacer 11. The right side face of the spacer 11 is opened so as to be capable of inhaling air. This opened part is the air inlet 42. And the left side face of the spacer 11 is of course opened to form a connection face to the connecting passage 41.

As a material for the moisture transmittable sheet 21, any of materials easily transmitting water vapor through them may be used and for example, a high-density cloth or a general cloth can be used. A high-density cloth in this case refers to cloth woven using about 300 lines of yarn per centimeter. And as the moisture transmittable sheet 21, it is desirable to use a material being small in thermal resistance. The reason for this is as follows. That is to say, a dehumidifying cushion provides a dehumidifying effect due to a fact that the moisture exhaled from the hips of a person being seated on a moisture transmittable sheet 21 is transmitted through the moisture transmittable sheet 21 and is carried off by air flowing through the dehumidifier air passage 10. At this time, it is possible to realize a sharp temperature gradient in the vicinity of the surface of the hips by making the outside air being lower in temperature than a body temperature flow through the dehumidifier air passage 10. Since it is possible to increase the quantity of heat emitted from the surface of the body due to this sharp temperature gradient, in the said dehumidifying cushion a secondary effect of cooling the hips can be also expected in addition to a dehumidifying effect. In a dehumidifying cushion aiming at not only dehumidifying but also cooling, therefore, a cooling effect as described above can be easily obtained by making it easy for heat to be transmitted from the hips to the dehumidifier air passage 10 through reducing the thermal resistance of the moisture transmittable sheet 21.

In case of applying a dehumidifier air passage 10 to a dehumidifying cushion aiming at only dehumidification, the quantity of air flowing through the dehumidifier air passage 10 may be less in comparison with the case of applying it to a dehumidifying cushion aiming at both of dehumidification and cooling. In such a case therefore there is no problem even if the resistance which air flowing through a dehumidifier air passage 10 receives from a spacer 11 is somewhat high.

As shown in Fig.1, the connecting passage 41 is a space connected to the dehumidifier air passage 10 and is attached to one of side faces of the dehumidifier air passage 10. The air outlet 43 for taking out air within the connecting passage 41 to the outside is provided in a nearly middle lower part of the connecting passage 41. The DC fan 44 is installed in a boundary area between the air outlet 43 and the connecting passage 41. This DC fan 44 generates forcibly the flow of air within the dehumidifier air passage 10 and the connecting passage 41. When the DC fan is driven, the outside air flows through the air inlet 42 into the dehumidifier air passage 10, passes through the dehumidifier air passage 10 and reaches the connecting passage 41. And this air is discharged by the DC fan 44 through the air outlet 43 to the outside.

The battery 45 is a power source for driving the DC fan 44. Such a battery 45 may be an ordinary dry cell but preferably it is a chargeable secondary battery so as to be capable of being charged by a commercial power supply when the dehumidifying cushion is not used.

The pressure switch 52 is provided in a nearly middle upper part of the dehumidifier air passage 10. The main switch 51 and the pressure switch 52 are connected in series with each other, and when both of them are on, electric power is supplied from the battery 45 to the DC fan 44.

In case of using a dehumidifying cushion, the dehumidifying cushion is placed on a chair so that the connecting passage 41 is located at the rear side (the back side) of the chair. Ordinarily, a humidifying cushion is placed so that the air outlet 43 faces downward, but it may be used upside down depending on the structure of a chair. When a person is seated on the dehumidifying cushion in this state, the pressure switch 52 detects the seating to be turned on. Further, when the main switch 51 has been turned on, the DC fan 44 is rotated so as to inhale the outside air through the air inlet 42. The inhaled air flows through the dehumidifier air passage 10 and then is discharged by the DC fan 44 through the air outlet 43 to the outside.

In such a dehumidifying cushion, the moisture exhaled from the surface of hips is transmitted through the moisture transmittable sheet 21 and enters the dehumidifier air passage 10. And the moisture which has entered is carried off to the outside by air flowing through the dehumidifier air passage 10. Due to this, when a person is seated on a dehumidifying cushion, the person can comfortably spend his or her time without a fact that the hips become moist due to sweat exhaled from the hips.

A dehumidifier air passage of the first embodiment uses a spacer which has a nearly flat base and a plurality of projections and is manufactured by cutting a foamed plastic material or by molding a foamed plastic material. Due to this, such a dehumidifier air passage is lightweight and flexible. And since a spacer can be easily manufactured, such a dehumidifier air passage is excellent in mass productivity. Accordingly, a dehumidifier air passage of the first embodiment is suitable for various kinds of dehumidifiers carrying off the moisture, which has been transmitted from the surface of a human body through a moisture transmittable sheet into a spacer, by means of air flowing through the spacer.

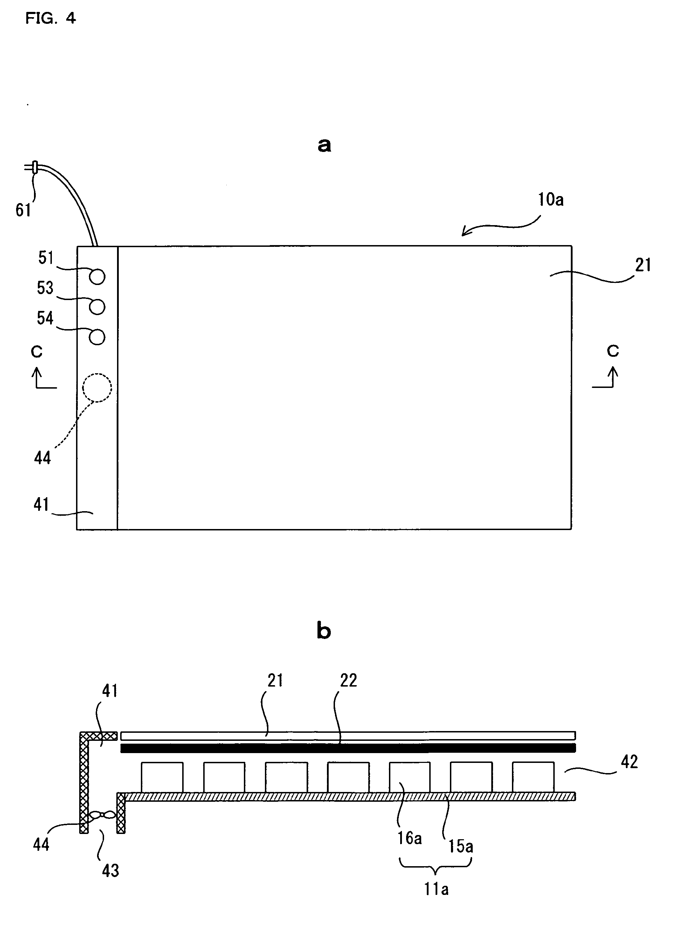

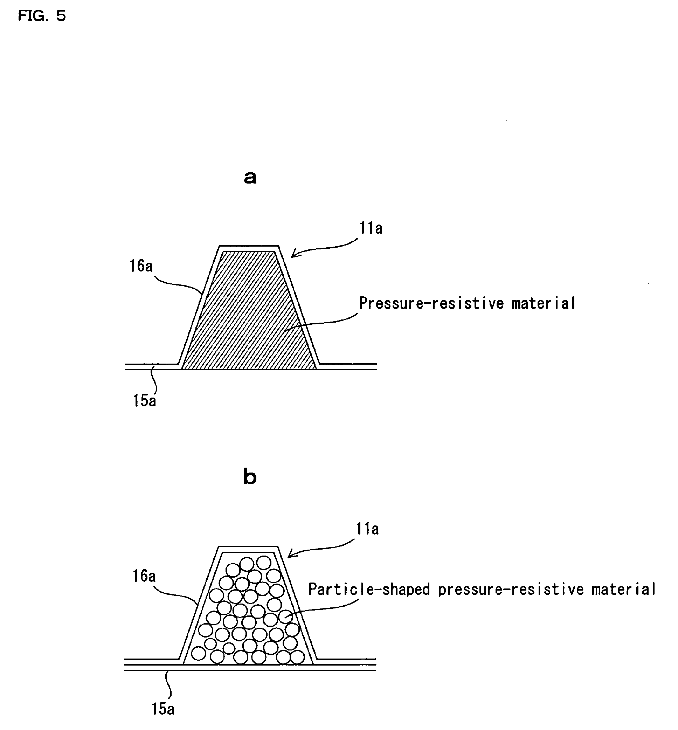

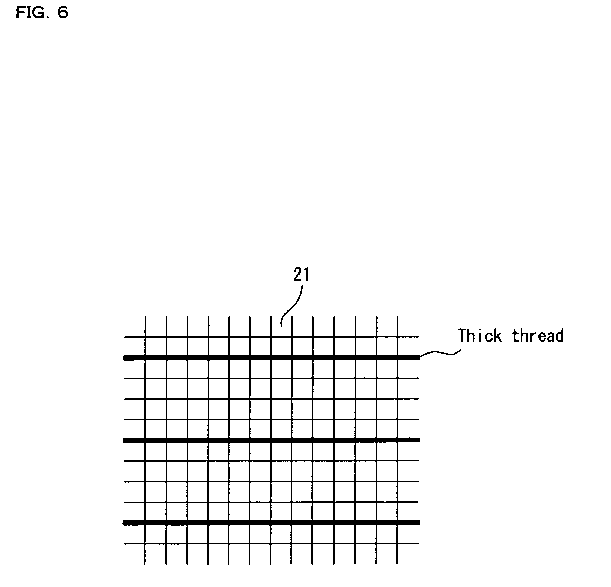

Next, a second embodiment of the present invention is described with reference to the drawings. Fig. 4a is a schematic plan view of a dehumidifying mat being a dehumidifier to which a dehumidifier air passage according to the second embodiment of the present invention is applied, Fig. 4b is a schematic sectional view of the dehumidifying mat seen from the direction of arrow C-C, Fig. 5 is a figure showing an example of a spacer to be used in a dehumidifier air passage according to the second embodiment, and Fig. 6 is a figure showing an example of a moisture transmittable sheet and a force distributing means to be used in a dehumidifier air passage according to the second embodiment. In the second embodiment, things having the same functions as those of the first embodiment are given the same symbols or corresponding symbols and detailed description of them is omitted.

A dehumidifying mat shown in Fig. 4 is used as a dehumidifier for removing the moisture coming out from the surface of the body when a person is lying on it. In the second embodiment, the case of using such a dehumidifying mat being placed on a bed is considered. Such a dehumidifying mat can be also used being placed on a sofa or a floor.

As shown in Fig. 4, this dehumidifying mat comprises a dehumidifier air passage 10a being nearly in the shape of a rectangle, a connecting passage 41, an air inlet 42, an air outlet 43, a DC fan 44 as a blowing means, a main switch 51, a flow rate adjusting knob 53, a timer 54 and a plug 61. And the dehumidifier air passage 10a has a nearly flat spacer 11a, a moisture transmittable sheet (moisture transmittable member) 21 and a force distributing means 22.

The spacer 11a is used for supporting the moisture transmittable sheet 21 and securing a space through which air flows. A plan view and a side view of this spacer 11a are nearly the same as those shown in Fig. 2 of the first embodiment. This spacer 11a has a nearly flat base 15a and a plurality of long and narrow projections 16a. Each of the projections 16a is arranged regularly so that the longitudinal direction of it is directed to a specified direction. These projections 16a are physically and continuously connected to one another through the nearly flat base 15a. The space between adjacent projections 16a becomes a space through which air flows. Thus, such a spacer 11a is also of a divided rail type similarly to the spacer in the first embodiment. And in the second embodiment also, similarly to the first embodiment, the projections 16a are formed so that the shape obtained by cutting them with a plane perpendicular to the longitudinal direction of them is a nearly tapered shape or a nearly corrugated shape.

In the second embodiment, a plastic film is used as a material for the base 15a. Concretely, a polyethylene film of several ten microns in thickness is used. Generally it is desirable that the thickness of this base 15a is 1 mm or less. And the inside of each projection 16a is stuffed up with a pressure-resistive material capable of confronting a pressure from the outside. A material being less in specific gravity in comparison with a material for the base 15a is used as this pressure-resistive material. For example, a foamed plastic material is used. Due to this, such a spacer 11a is characterized by being lightweight and flexible and furthermore being inexpensive.

This spacer 11a can be manufactured in a manner as described below. First, a plastic film is placed on a metal mold having depressions corresponding to the projections 16a. And a plurality of depressions are formed in the plastic film by vacuum-molding the plastic film as heating the metal mold. These depressions become the projections 16a later. Next, the depressions of the plastic film are stuffed up with an adhesive pressure-resistive material for example. Thus, a spacer 11a can be easily manufactured. A spacer 11a manufactured by such a method is shown in Fig. 5a. Hereupon, Fig. 5 is a schematic sectional view of part of a spacer 11a obtained by cutting the spacer 11a with a plane perpendicular to the longitudinal direction of the projections 16a. And a pressure-resistive material in the shape of small particles may be used in place of an adhesive pressure-resistive material. In this case, after the depressions of the plastic film are stuffed up with a pressure-resistive material in the shape of particles, the reverse face of the plastic film is laminated with another plastic film so that the pressure-resistive material in the shape of particles does not fly away. A spacer 11a manufactured by such a method is shown in Fig. 5b. A spacer 11a may be continuously manufactured by molding as turning a metal mold formed into the shape of a roll.

The force distributing means 22 and the moisture transmittable sheet 21 are provided in this order over the projections 16a of the spacer 11a. The moisture transmittable sheet 21 is nearly the same as that of the first embodiment and wraps the obverse face and the upper and lower side faces of the spacer 11a in it in Fig. 4a. The force distributing means 22 is used for distributing and applying a force applied from a person lying down on the moisture transmittable sheet 21 to the spacer 11a. As the force distributing means 22, for example a meshed material is used. In general, a force distributing means 22 does not necessarily need to be meshed but may be any material being flexible, small in thermal resistance and excellent in moisture transmittance.

And by using this force distributing means 22, it is possible to suppress a rugged feeling caused by pressing of the projections 16a of the spacer 11a against the body and prevent parts of the moisture transmittable sheet 21 coming in between the projections 16a of the spacer 11a from hindering the flow of air. It is desirable also to provide a force distributing means in a dehumidifier air passage of the above-mentioned first embodiment.

In the second embodiment, such a force distributing means 22 and a moisture transmittable sheet 21 are formed into one body. In order to form the moisture transmittable sheet 21 and the force distributing means 22 into one body, it is enough to laminate the force distributing means 22 on the reverse face of the moisture transmittable sheet 21. Or in case of making cloth for the reverse face of the moisture transmittable sheet 21, it is enough to knit thick threads for structure into the said cloth at regular intervals along the longitudinal and/or lateral direction. Hereupon, whether threads for structure are to be knitted in the shape of meshes or at regular intervals along one direction may be determined according to the shape of the projections 16a of the spacer 11a. For example, in case that each projection 16a is long and narrow, threads for structure are knitted in the reverse face of a moisture transmittable sheet 21 at regular intervals along one direction as shown in Fig.6. And the moisture transmittable sheet 21 is placed on the spacer 11a so that the knitting direction and the longitudinal direction of the projections 16a are perpendicular to each other. The force distributing means 22 and the moisture transmittable sheet 21 can be easily formed into one body by means of such a method.

The connecting passage 41, the air inlet 42, the air outlet 43, the DC fan 44 and the main switch 51 are nearly the same as those of the first embodiment.

The flow rate adjusting knob 53 is used for adjusting the quantity of air flowing through the dehumidifier air passage 10a by varying the speed of rotation of the DC fan 44. The timer 54 is used for setting the time of automatically stopping the operation of the DC fan 44. The plug 61 is used for connecting the dehumidifying mat to a commercial power supply.

In case of using a dehumidifying mat, ordinarily the dehumidifying mat is placed on a bed so that the air outlet 43 faces downward. When the main switch 51 is turned on, the DC fan 44 is rotated so as to inhale the outside air through the air inlet 42. The inhaled air flows through the dehumidifier air passage 10a and then is discharged by the DC fan 44 through the air outlet 43 to the outside.

In such a dehumidifying mat, the moisture exhaled from the surface of a human body is transmitted through the moisture transmittable sheet 21 and comes into the dehumidifier air passage 10a. And the moisture which has come in is carried off to the outside by air flowing through the dehumidifier air passage 10a. Due to this, when a person lies on a dehumidifying mat, he or she can comfortably spend his or her time without a fact that his or her body becomes moist with perspiration due to sweat exhaled from the human body.

A dehumidifier air passage of the second embodiment uses a spacer which has a nearly flat base and a plurality of projections and is manufactured by forming a plurality of depressions in a plastic film by means of a molding method and then stuffing up each of the depressions with a pressure-resistive material. By using a foamed plastic material as the pressure-resistive material in this case, it is possible to make such a dehumidifier air passage more lightweight and flexible. And such a dehumidifier air passage is suitable for mass production and very excellent in cost performance. The problem of cost is important particularly in case of forming a large-size dehumidifier air passage. Accordingly, a dehumidifier air passage of the second embodiment is suitable for the use in a dehumidifier for a mat, a mattress, bed and the like for example where a large-size dehumidifier air passage needs to be formed. A spacer in such a dehumidifier air passage can bear a heavy load. Therefore, when a person stands on a dehumidifier using the said air passage, the said air passage is not broken.

And in a dehumidifier air passage of the second embodiment, forming a force distributing means and a moisture transmittable sheet into one body makes it easy to manufacture and assemble the dehumidifier air passage.

In the second embodiment described above, a plurality of holes may be made in the base 15a of the spacer 11a according to need. In case that a DC fan 44 forces air to flow through a spacer 11a there is no problem, but in case that the DC fan 44 is at a stop, the inside of the spacer 11a may become moist. In such a case, it is possible to make the moisture go out to the outside by providing holes.

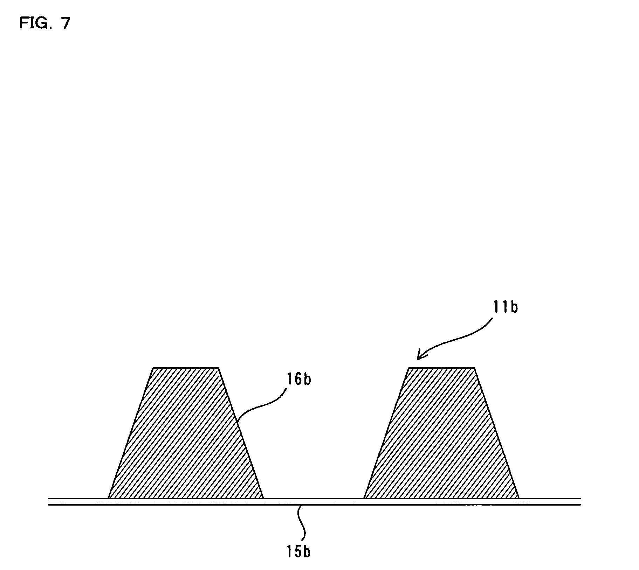

And in the second embodiment described above, although there has been described the case of using a spacer manufactured by forming a plurality of depressions in a plastic film by means of a molding method and then stuffing up each of the depressions with a pressure-resistive material, various spacers other than this spacer can be used. Fig. 7 is a figure for explaining another spacer to be used in a dehumidifier air passage according to the second embodiment.

A spacer 11b shown in Fig. 7 has a nearly flat base 15b and a plurality of long and narrow projections 16b, and is obtained by joining the plurality of projections 16b onto the base 15b. A plan view and a side view of this spacer 11b are nearly the same as those shown in Fig. 2 of the first embodiment. And Fig. 7 is a schematic sectional view of part of the spacer 11b obtained by cutting the said spacer 11b with a plane perpendicular to the longitudinal direction of the projections 16b.

In addition to a plastic film, cloth or meshed materials can be used as a material for the base 15b. And a pressure-resistive material being different from a material for the base 15b and capable of confronting a pressure from the outside is used as a material for the projections 16b. That is to say, a material being smaller in specific gravity in comparison with a material for the base 15b, for example, a foamed plastic material is used as a pressure-resistive material. Due to this, such a spacer 11b is characterized by being lightweight and flexible and furthermore being inexpensive similarly to the spacer shown in Fig. 5.

This spacer 11b can be manufactured in a manner as described below. First, a metal mold having depressions corresponding to projections 16b is heated and a foamed plastic material is poured into the depressions. And the foamed plastic material adhering to other parts than the depressions is removed by means of a spatula or the like. Next, a nearly flat base 15b is placed on the metal mold and the foamed plastic material contained in the depressions is made to adhere to the base 15b. Hereupon, in case of using a plastic film as the base 15b, since the foamed plastic material adheres to the plastic film, an adhesive agent is not necessary. And in case of using cloth or a meshed material as the base 15b, a liquefied foamed plastic material soaks into cloth or the like, and thereby the base 15b and the projections 16b can be formed into one body. In such a manner a spacer 11b can be easily manufactured. The spacer 11b can be continuously manufactured by making the base 15b and the foamed plastic material adhere to each other as rotating a metal mold formed in the shape of a roll.

A dehumidifier air passage manufactured using such a spacer 11b provides a similar effect to that of the second embodiment described above.

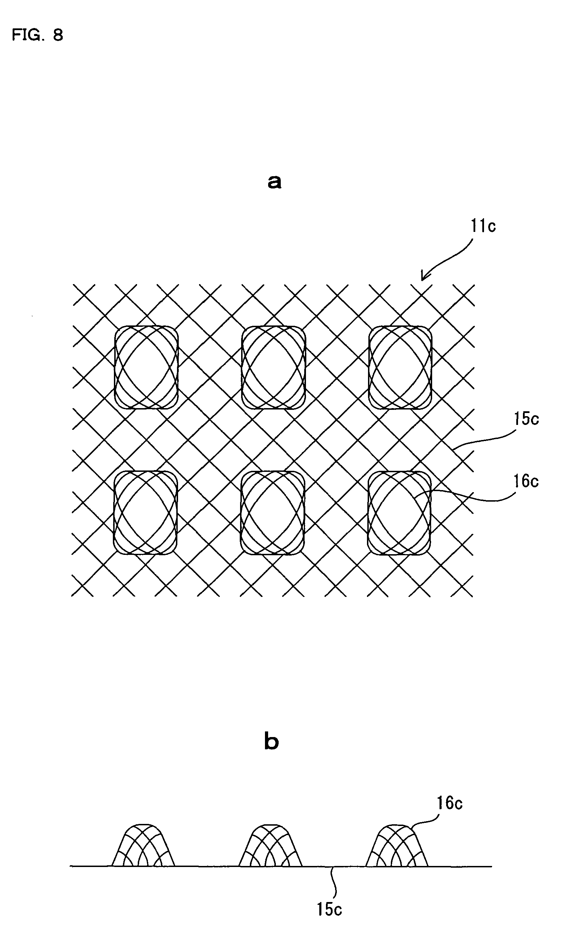

And another spacer is described. Fig. 8a is a schematic plan view of another spacer to be used in a dehumidifier air passage according to the second embodiment, and Fig. 8b is a schematic side view of this spacer.

A spacer 11c shown in Fig. 8 has a nearly flat base 15c and a plurality of projections 16c. Each of the projections 16c is arranged regularly so that the longitudinal direction of it is directed to a specified direction. And the spacer 11c is obtained by molding a meshed member. Due to this, such a spacer 11c is lightweight and flexible. Further, the resistance which air flowing through the spacer 11c receives from the projections 16c is a little and the air can smoothly flow around the projections 16c. Therefore, if the meshes of a meshed member are not so small in mesh size, the density of forming the projections 16c can be made large. This is a characteristic point of the said spacer 11c not found in other spacers.

A plastic material for example can be used as a meshed member being a material for a spacer 11c. In this case the spacer 11c can be manufactured by thermal press-forming. That is to say, a meshed member is first placed between two metal molds. And the spacer 11c can be easily manufactured by heating and pressing the two metal molds against each other.

A dehumidifier air passage manufactured using such a spacer 11c provides a similar effect to that of the second embodiment described above.

It might be feared that in a dehumidifier air passage having such a spacer 11c, in case of providing no cloth or the like under the spacer 11c, air flowing through the spacer 11c may leak to the outside from there. However, since a dehumidifier air passage is ordinarily installed so that its bottom is placed on a floor or the like, there is no problem if the bottom of the spacer 11c is not covered with cloth or the like.

Although there has been described the case of forming a force distributing means and a moisture transmittable sheet into one body in the second embodiment described above, the force distributing means and the moisture transmittable sheet do not necessarily need to be formed into one body. And a meshed member may be used as a force distributing means and this meshed member may be joined to the projections of a spacer so as not to slacken. In this case, a thin and soft material to be used in a screen door and the like for example is used as a material for the meshed member. This meshed member is joined to the projections of the spacer by means of an adhesive agent and the like so as not to slacken. Due to this, since the meshed member can keep its shape when a thing is placed on it, it can perform its role as a force distributing means.

And a moisture transmittable sheet may have a role as a force distributing means. That is to say, for example, a moisture transmittable sheet may be joined to the projections of a spacer so as not to slacken without providing an independent force distributing means. In this case, the moisture transmittable sheet is joined to the projections by means of an adhesive agent and the like. Due to this, since the moisture transmittable sheet can keep its shape when a thing is placed on it, it can perform its role as a force distributing means.

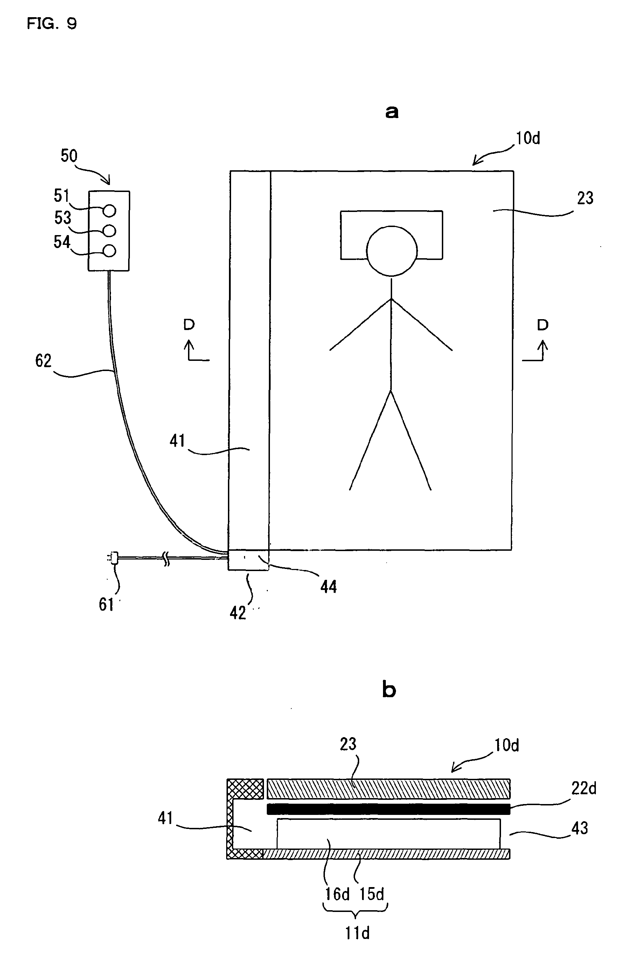

Next, a third embodiment of the present invention is described with reference to the drawings. Fig. 9a is a schematic plan view of a dehumidifying mattress being a dehumidifier to which a dehumidifier air passage according to the third embodiment of the present invention is applied, Fig. 9b is a schematic sectional view of the dehumidifying mattress seen from the direction of arrow D-D, Fig. 10a is a schematic plan view of a spacer to be used in a dehumidifier air passage according to the third embodiment, and Fig. 10b is a schematic side view of this spacer. In the third embodiment, things having the same functions as those of the first and second embodiments are given the same symbols or corresponding symbols and detailed description of them is omitted.

A dehumidifying mattress shown in Fig. 9 is used as a dehumidifier for removing the moisture coming out from the surface of the body when a person lies down on it. In the third embodiment, the case of using such a dehumidifying mattress for the purpose of only dehumidification is considered. That is to say, the use of it does not aim at cooling a human body.

As shown in Fig. 9, this dehumidifying mattress comprises a dehumidifier air passage 10d being nearly in the shape of a rectangle, a connecting passage 41, an air inlet 42, an air outlet 43, a DC fan 44 as a blowing means, a controller 50, a plug 61 and a cord 62.

The dehumidifier air passage 10d has a nearly flat spacer 11d, a force distributing means 22d and a moisture transmittable mat (moisture transmittable member) 23, as shown in Fig. 9. In Fig. 9a, the right side face of the dehumidifier air passage 10d is the air outlet 43 for discharging air within the said air passage 10d to the outside.

The spacer 11d is used for supporting the moisture transmittable mat 23 and securing a space through which air flows. This spacer 11d is of a continuous rail type, and has a nearly flat base 15d and a plurality of rail-shaped projections 16d, as shown in Figs. 9 and 10. The plurality of rail-shaped projections 16d are arranged at regular intervals so that the longitudinal direction of them is in parallel with the short side of the nearly flat base 15d. These projections 16d are physically and continuously connected to one another through the nearly flat base 15d. The space between adjacent projections 16d becomes a space through which air flows.

In the third embodiment, the spacer 11d is manufactured by extrusion-molding a polycarbonate material. That is to say, the spacer 11d is formed by pouring the heated and liquefied polycarbonate material into metal molds. The reason why an extrusion-molding process using metal molds can be performed is that any part of the spacer 11d along the direction of thickness of it does not overlap another part with a space between them.

A spacer 11d of a continuous rail type can bear a heavier load in comparison with a spacer of a divided rail type. Furthermore, polycarbonate is a very strong material. Therefore, a spacer 11d of a continuous rail type made of polycarbonate is characterized by being very excellent in strength. Due to this, the spacer 11d shown in Fig. 10 is suitable for being used in a dehumidifier such as a mattress and the like.

A force distributing means 22d is provided over the projections 16d of the spacer 11d. The force distributing means 22d is used for distributing and applying a force applied from a person when he or she lies down on a moisture transmittable mat 23 to the spacer 11d. Since such a force distributing means 22d is provided between the spacer 11d and the moisture transmittable mat 23, a material being excellent in moisture transmittance needs to be used as the force distributing means 22d so that moisture can be transmitted between them. And since the spacer 11d supports a thick moisture transmittable mat 23 in the third embodiment, a material being excellent in strength needs to be used as the force distributing means 22d. Considering these points, it is desirable to use, for example, a strong meshed member or a plastic plate having a number of small holes made in it as the force distributing means 22d. In the third embodiment, the force distributing means 22d does not necessarily need to be flexible.

A moisture transmittable mat 23 is used for a person to lie down on it and is placed on a force distributing means 22d. A material being 2 mm or more in thickness and flexible is used as this moisture transmittable mat 23. It is possible to improve one's feeling in bed by using such a moisture transmittable mat 23.

By the way, generally in a dehumidifier aiming at not only dehumidifying but also cooling a human body, it is desirable to use a moisture transmittable mat being as thin and small as possible in thickness and thermal resistance. The reason is that heat from a human body can be easily moved into a dehumidifier air passage. On the other hand, in a dehumidifier aiming at only dehumidification, since cooling does not need to be considered, it is desirable to use a moisture transmittable mat having a large thermal resistance, namely, a thermal insulating ability. Due to this, in the third embodiment, a moisture transmittable mat having a thermal insulating ability is used as a moisture transmittable mat 23. The thermal insulating ability of a moisture transmittable mat 23 is generally determined by the quantity of air contained by the moisture transmittable mat 23.

Now, the quantity of air per area of 1 cm² in a plane nearly perpendicular to the direction of thickness (height) of a spacer 11d when a person mounts the moisture transmittable mat 23 to apply a pressure to the mat 23 is considered as the air content of a moisture transmittable mat 23. If the air content of the moisture transmittable mat 23 is at least 0.05 cc per the said area of 1 cm², the moisture transmittable mat 23 is considered to have a sufficient thermal insulating ability. Particularly, if the air content of the moisture transmittable mat 23 is 0.1 cc per the said area of 1 cm², the moisture transmittable mat 23 can be said to have an almost complete thermal insulating ability. Hereupon, that the air content of the moisture transmittable mat 23 is 0.1 cc per the said area of 1 cm² is nearly equal to a fact that a thermal insulation is performed by an air layer of 1 mm in thickness.

A moisture transmittable sheet may be provided between the spacer 11d and the moisture transmittable mat 23 similarly to the first embodiment described above.

The connecting passage 41 is a space connected to the dehumidifier air passage 10d and is provided at the left side of the dehumidifier air passage 10d in Fig. 9a. The DC fan 44 is installed at the lower end of the connecting passage 41. An opening of the portion at which the DC fan 44 is installed becomes the air inlet 42 for taking the outside air into the connecting passage 41. The DC fan 44 forcibly generates the flow of air within the dehumidifier air passage 10d and the connecting passage 41. When the DC fan 44 is driven, the outside air flows through the air inlet 42 into the connecting passage 41, passes through the connecting passage 41 and reaches the dehumidifier air passage 10d. And this air is discharged through the air outlet 43 to the outside.

A controller 50 is connected through the cord 62 to the DC fan 44. This controller 50 has the main switch 51, the flow rate adjusting knob 53 and the timer 54. The main switch 51, the flow rate adjusting knob 53 and the timer 54 are the same as those of the second embodiment. And the plug 61 is used for connection with a commercial power supply.

In case of using a dehumidifying mattress, when the main switch 51 is turned on, the DC fan 44 is rotated so as to inhale the outside air through the air inlet 42. The inhaled air flows through the connecting passage 41 and then comes into the dehumidifier air passage 10d. And this air flows from the right side to the left side of a lying person and is discharged through the air outlet 43 to the outside.

When a person lies down on a moisture transmittable mat 23, moisture such as sweat and the like is exhaled from the human body. And the moisture transmittable mat 23 becomes moist due to such moisture. This moisture can be transmitted into the dehumidifier air passage 10d and carried off by air flowing through the dehumidifier air passage 10d to the outside. Due to this, moisture of the moisture transmittable mat 23 can be removed and therefore, a lying person can pass his or her comfortable time without becoming moist due to sweat exhaled from the human body. And such a dehumidifying mattress is preferable in being used in a mattress to be a so-called long unmade bed in case that a person lies in a sickbed. Due to this, since a human body does not become moist, it is possible to prevent bedsores from forming.

For example, a cushion material may be provided under the base 15d of the spacer 11d in order to make the dehumidifier air passage 10d flexible.

A dehumidifier air passage of the third embodiment uses a spacer which has a nearly flat base and a plurality of rail-shaped projections formed on the base so as to be in parallel with one another and is manufactured by means of an extrusion molding method. Due to this, such a dehumidifier air passage can bear a heavy load. Therefore, a dehumidifier air passage of the third embodiment is particularly suitable for being used in a dehumidifier for a mattress and the like. And this dehumidifier air passage is excellent in mass productivity.

And in the third embodiment, since a moisture transmittable mat having the air content being at least 0.05 cc per area of 1 cm² in a plane perpendicular to the direction of thickness of a spacer is used as a moisture transmittable mat, this moisture transmittable mat is excellent in thermal insulation. Therefore, a dehumidifier air passage having such a moisture transmittable mat is preferable for being used in a dehumidifier aiming at only dehumidification.

The present invention is not limited to the above-mentioned embodiments but enables various variations and combinations within the scope of its purport.

Although there have been described the cases that a dehumidifier air passage of the present invention is applied to a dehumidifying cushion, a dehumidifying mat and a dehumidifying mattress in the above-mentioned embodiments, in addition to these it is possible also to apply the present invention to, for example, a dehumidifying chair the seat of which is provided with the said air passage, dehumidifying clothes the reverse face of which is provided with the said air passage on it, a dehumidifying shoe the sole of which is provided with the said air passage, a dehumidifying pillow provided with the said air passage, and the like.

Hereupon, dehumidifying clothes are described as an example. In such dehumidifying clothes, a dehumidifier air passage is attached to the reverse face of a clothes material. This dehumidifier air passage comprises a nearly flat spacer for securing a space in which air flows nearly in parallel with the surface of body between a human body or underwear and a clothes material. Such dehumidifying clothes can dehumidify a wearer by carrying off the moisture which has come into a dehumidifier air passage from the surface of a human body by means of air flowing through this air passage.

And in the above-mentioned embodiments, the strength of pressure resistance of a spacer is determined depending on the purpose of use and the like of a dehumidifier to which the said dehumidifier air passage is applied. For example, in case that the said dehumidifier air passage is applied to a mattress, a spacer being capable of bearing the weight of a person is used. And in case that the said dehumidifier air passage is applied to clothes, since a spacer is not subject to a heavy load, it is enough to use a spacer capable of bearing a small force. Due to this, in case of dehumidifying clothes, a spacer results in attaching more importance to its light weight than its strength of pressure resistance. Concretely, it is preferable to use a spacer shown in Fig. 5, 7 or 8 as a spacer to be applied to dehumidifying clothes.

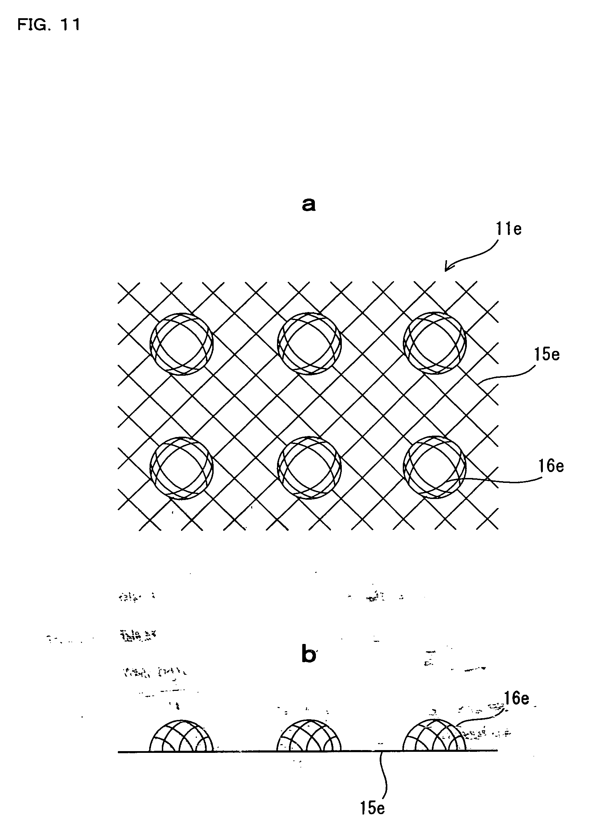

In case of applying a dehumidifier air passage to dehumidifying clothes, it is particularly desirable to use a spacer shown in Fig. 11. Fig. 11a is a schematic plan view of a spacer to be used in an air passage for dehumidifying clothes and Fig. 11b is a schematic side view of this spacer.

A spacer 11e shown in Fig. 11 has nearly the same structure as the spacer shown in Fig. 8, and has a nearly flat base 15e and a plurality of projections 16e. Hereupon, each of the projections 16e is formed nearly in the shape of a semicircle. This spacer 11e is manufactured by molding a meshed member.

Cloth is used as a meshed member being a material for the spacer 11e. And plastic is impregnated into parts of cloth corresponding to the projections 16e. In order to manufacture this spacer 11e, meshed cloth is first put in a specific mold. And the spacer 11e can be manufactured by pouring plastic into only the parts of the mold corresponding to the projections. Thus, such a spacer 11e is particularly excellent in flexibility due to its base 15e made of cloth.

Although the case that a plurality of projections protrude on only one side of the base has been described in the spacer shown in Fig. 11, a plurality of projections may protrude on both sides of the base.

Although the case that a dehumidifier air passage of the present invention is applied to a dehumidifier for removing moisture exhaled from a human body has been described in the above-mentioned embodiments, it can be also applied to a dehumidifier for removing moisture exhaled from an animal other than a human being or a dehumidifier for removing moisture exhaled from a plant.

As described above, in a dehumidifier air passage according to the present invention, by using a spacer having a nearly flat base and a plurality of projections as a spacer, it is possible to make a sufficient amount of air flow through the spacer. Due to this, a sufficient effect of dehumidification can be obtained in case of applying such a dehumidifier air passage to a dehumidifying cushion, a dehumidifying mat, a dehumidifying mattress, dehumidifying clothes and the like. And since such a spacer can be easily manufactured by a molding method and the like, a dehumidifier air passage of the present invention is excellent in mass productivity.

### INDUSTRIAL APPLICABILITY

As described above, the present invention can be applied to a dehumidifier such as a dehumidifying cushion, a dehumidifying mat, a dehumidifying mattress, dehumidifying clothes and the like for removing moisture by making the outside air flow in the vicinity of the surface of a human body and the like.

## Claims

1. A dehumidifier air passage to be used in a dehumidifier, comprising a moisture transmittable member provided at a side to be in contact with a living thing and a nearly flat spacer for supporting said moisture transmittable member and for securing a space through which air flows, said dehumidifier carrying off the moisture transmitted into said space through said moisture transmittable member from the surface of said living thing by means of air flowing through said space, wherein
said spacer has a nearly flat base and a plurality of projections and is obtained by cutting a foamed plastic material or by molding a foamed plastic material, and said dehumidifier air passage has a force distributing means for distributing and applying a force applied from said living thing to said spacer, said force distributing means being provided between said moisture transmittable member and said spacer.

2. A dehumidifier air passage to be used in a dehumidifier, comprising a moisture transmittable member provided at a side to be in contact with a living thing and a nearly flat spacer for supporting said moisture transmittable member and for securing a space through which air flows, said dehumidifier carrying off the moisture transmitted into said space through said moisture transmittable member from the surface of said living thing by means of air flowing through said space, wherein
said spacer has a nearly flat base and a plurality of projections and is obtained by forming a plurality of depressions in a plastic film by means of a molding method and then stuffing up each of said depressions with a pressure-resistive material.

3. A dehumidifier air passage to be used in a dehumidifier, comprising a moisture transmittable member provided at a side to be in contact with a living thing and a nearly flat spacer for supporting said moisture transmittable member and for securing a space through which air flows, said dehumidifier carrying off the moisture transmitted into said space through said moisture transmittable member from the surface of said living thing by means of air flowing through said space, wherein
said spacer has a nearly flat base and a plurality of projections and is obtained by joining said plurality of projections onto said base.

4. A dehumidifier air passage to be used in a dehumidifier, comprising a moisture transmittable member provided at a side to be in contact with a living thing and a nearly flat spacer for supporting said moisture transmittable member and for securing a space through which air flows, said dehumidifier carrying off the moisture transmitted into said space through said moisture transmittable member from the surface of said living thing by means of air flowing through said space, wherein
said spacer is obtained by molding a meshed member.

5. A dehumidifying clothes air passage to be used in dehumidifying clothes, comprising a nearly flat spacer for securing a space through which air flows between a human body or underwear and a clothes material, said dehumidifying clothes carrying off the moisture which has come into said space from the surface of said human body by means of air flowing through said space, wherein
said spacer is obtained by molding a meshed member.

6. A dehumidifier air passage according to claim 2, 3 or 4, comprising a force distributing means for distributing and applying a force applied from said living thing to said spacer, said force distributing means being provided between said moisture transmittable member and said spacer.

7. A dehumidifier air passage according to claim 1 or 6, wherein said force distributing means is formed into one body together with said moisture transmittable member.

8. A dehumidifier air passage according to claim 2 or 3, wherein a meshed material is provided between said spacer and said moisture transmittable member, and said meshed material is joined to said plurality of projections so as not to slacken.

9. A dehumidifier air passage according to claim 1, wherein said force distributing means is joined to said plurality of projections so as not to slacken.

10. A dehumidifier air passage according to claim 2 or 3, wherein said moisture transmittable member is joined to said plurality of projections so as not to slacken.

11. A dehumidifier air passage according to claim 1, 2 or 3, wherein each of said projections is formed so that the face of it facing said moisture transmittable member is at most 50 % in area of the face being in contact with said base.

12. A dehumidifier air passage to be used in a dehumidifier, comprising a moisture transmittable mat provided at a side to be in contact with a living thing and a nearly flat spacer for supporting said moisture transmittable mat and for securing a space through which air flows, said dehumidifier carrying off the moisture transmitted into said space through said moisture transmittable mat from the surface of said living thing by means of air flowing through said space, wherein
said spacer has a nearly flat base and a plurality of rail-shaped projections formed in parallel with one another on said base and is obtained by an extrusion molding method, and said dehumidifier air passage has a force distributing means for distributing and applying a force applied from said living thing to said spacer, said force distributing means being provided between said spacer and said moisture transmittable mat.

13. A dehumidifier air passage to be used in a dehumidifier, comprising a moisture transmittable mat provided at a side to be in contact with a living thing and a nearly flat spacer for supporting said moisture transmittable mat and for securing a space through which air flows, said dehumidifier carrying off the moisture transmitted into said space through said moisture transmittable mat from the surface of said living thing by means of air flowing through said space, wherein
said moisture transmittable mat has a thermal insulation ability and the air content of said moisture transmittable mat is at least 0.05 cc per area of 1 cm² in a plane nearly perpendicular to the direction of thickness of said spacer.

14. A dehumidifier air passage according to claim 13, comprising a force distributing means for distributing and applying a force applied from said living thing to said spacer, said force distributing means being provided between said spacer and said moisture transmittable mat.

15. A dehumidifier air passage according to claim 13 or 14, comprising a cushion material provided under said spacer.
